# EUROPÄISCHE PATENTSCHRIFT

(11) **EP 1 165 025 B1**
(45) Veröffentlichungstag und Bekanntmachung des Hinweises auf die Patenterteilung: **21.01.2004**
(21) Anmeldenummer: 01948933.5
(22) Anmeldetag: 30.01.2001
(51) Int. Cl.: A61K 7/06, A61K 7/11, A61K 7/00

(54) **KOMPAKTHAARSPRAYPRODUKT BESTEHEND AUS KONZENTRAT, BEHÄLTER UND EINER FEINSPRÜHPUMPE MIT VORDRUCKAUFBAU**
COMPACT HAIRSPRAY PRODUCT CONSISTING OF HAIRSPRAY CONCENTRATE, CONTAINER AND FINE SPRAY PUMP WITH PRE-PRESSURIZATION
PRODUIT COMPACT POUR LAQUE CAPILLAIRE CONSTITUE D'UN CONCENTRE, D'UN RECIPIENT ET D'UNE POMPE AVEC PRECOMPRESSION ET A VAPORISATION FINE

(30) Priorität: 03.02.2000 DE 10004769
(43) Veröffentlichungstag der Anmeldung: 02.01.2002
(73) Patentinhaber: Wella Aktiengesellschaft, 64274 Darmstadt (DE)
(72) Erfinder: STEINMETZ, Uwe, 64274 Reinheim (DE); STARKE, Thomas, 64367 Muehltal (DE); STEIGERWALD, Franz, 64347 Griesheim (DE)
(74) Vertreter: Schulze, Rainer (DE)
(86) Internationale Anmeldenummer: PCT/EP2001/000958
(87) Internationale Veröffentlichungsnummer: WO 2001/056536

(56) Entgegenhaltungen:
- DE-A- 3 941 572
- US-A- 5 032 387
- US-A- 5 626 835

## Beschreibung

Gegenstand der Erfindung ist ein Kompakthaarsprayprodukt bestehend aus einem Haarspraykonzentrat, einem druckfesten und diffusionsdichten Behälter und einer Feinsprühpumpe mit Vordruckaufbau.

Haarsprayprodukte werden üblicherweise entweder als in Aerosolbehältern abgefüllte, Treibgase enthaltende Aerosol-Sprays angeboten oder als treibgasfreie sogenannte Pumpsprays, welche mittels einer mechanischen Sprühpumpe versprüht werden,wobei die Pumphubvolumina üblicherweise 120-190 µl betragen. Dabei kommt den Aerosol-Sprays aufgrund ihrer besseren Performance und ihren besseren Anwendungseigenschaften eine wesentlich größere Bedeutung zu. Die klassischen Aerosolhaarsprays haben aber den Nachteil, dass sie produktbedingt ein relativ großes und unhandliches Format aufweisen. Dies bedingt wiederum wenig attraktive und schlecht gestaltbare Verpackungsformen. Üblich sind z.B. 250ml-Dosen aus Weißblech oder Aluminium in klassischer Zylinderform. Das relativ große Format ist auf den vergleichsweise großen, keinen Wirkstoff enthaltenden, für das Treibmittel erforderlichen Gasraum zurückzuführen. Pumsprays kommen zwar ohne Treibmittel aus und es können daher im Vergleich zu Aerosoldosen wenig voluminösere Verpackungen verwendet werden, aber dafür müssen deutliche Einbußen bei den Anwendungseigenschaften in Kauf genommen werden. Die üblichen, bekannten Pumpsprays werden vom Anwender gegenüber den Aerosolsprays hauptsächlich als feuchter und gröber empfunden, sie haben eine längere Trocknungszeit und ein gröberes Tropfenspektrum. Der Marktanteil ist entsprechend gering. Darüberhinaus war es wegen der relativ geringen erreichbaren Konzentration an haarfestigenden Polymeren in Aerosolsprays und des hohen Kopfraumbedarfs bisher nicht möglich, kompakte Aerosol-Haarsprayprodukte herzustellen, die sowohl in den Anwendungseigenschaften als auch in der Ergiebigkeit an Aerosolsprays in den üblichen Packungsgrößen um 250 ml heranreichen.

Haarsprays werden üblicherweise in Form von Polymerlösungen in alkoholischem Milieu verwendet. Die Polymerkonzentration ist hierbei relativ begrenzt. Nach Schrader, 'Grundlagen und Rezepturen der Kosmetika', 2. Auflage, 1989, Seite 772, beträgt der typische Polymergehalt eines Haarsprays 2-4%. Ein erhöhter Polymergehalt führt zu vielfältigen Problemen, welche insbesondere auf die stark ansteigende Viskosität der Zusammensetzung zurückzuführen sind. So steigt beispielsweise die Tröpfchengröße der versprühten Zusammensetzung bei Erhöhung der Harzkonzentration auf üblicher, alkoholischer Lösungsmittelbasis unakzeptabel auf größer 100 µm an. Gute Pumphaarsprays haben eine mittlere Tröpfchengröße von ca. 80 µm, je kleiner (z.B. 50 µm) desto besser. Nachteile von zu großen Tropfen sind der sogenannte Nasseffekt, der sogenannte Perlenketteneffekt, bei dem sich auf dem Haar sichtbare, schlecht verlaufende und schlecht trocknende perlenartige Tröpfchen bilden sowie lange Trocknungszeiten, schlechte Verteilung auf dem Haar, ein verschlechtertes Sprühbild etc. Neben der mittleren Tröpfchengröße ist auch die Tröpfchengrößenverteilung ausschlaggebend für die Qualität eines Haarsprays, da bereits eine relativ geringe Anzahl von sehr großen Tropfen erhebliche Nachteile bringt. Ein Maß für die Tröpfchengrößenverteilung sind die dv(50) bzw. dv(90) Werte. Diese Werte geben den maximalen Durchmesser an, den 50% bzw. 90% aller Tröpfchen besitzen. Für typische Aerosolhaarsprays liegt der durchschnittliche dv(50) Wert bei ca. 40 µm und der durchschnittliche dv(90) Wert bei ca. 75µm. Bei typischen Pumphaarsprays liegt der durchschnittliche dv(50) Wert bei ca. 75 µm und der durchschnittliche dv(90) Wert bei ca. 120 µm. Insbesondere der dv(90) Wert sollte nicht über 140 µm, vorzugsweise nicht über 130 µm liegen. Ansonsten werden die Sprays als sehr nass empfunden.

Aus der EP 0 460 123 A ist ein einphasiges Lösungsmittelgemisch aus C2- bis C4-Alkoholen, Wasser und einem niederen Alkan, ausgewählt aus n-Pentan, n-Hexan und Isohexan bekannt. Dieses Lösungsmittelgemisch kann in Non-Aerosol Haarsprays eingesetzt werden. Haarspraykonzentrate und Kompakthaarsprays werden nicht beschrieben.

Wünschenswert sind daher kleine, kompakte Haarsprayprodukte, welche sowohl gegenüber üblichen Aerosol-Sprays als auch gegenüber üblichen Pumphaarsprays bei vergleichbarer Ergiebigkeit mit einem Bruchteil des Verpackungsvolumens auskommen, gleichzeitig aber in den Anwendungseigenschaften wie z.B. der Festigungsleistung und des Sprühverhaltens nicht die Nachteile der üblichen Pumphaarsprays aufweisen, sondern an die Eigenschaften der Aerosol-Sprays heranreichen und außerdem eine aus Umweltschutzgesichtspunkten wünschenswerte verbesserte VOC-Bilanz aufweisen.

Es wurde nun gefunden, dass die Aufgabe gelöst wird durch ein Kompakthaarsprayprodukt bestehend aus einem speziellen Haarspraykonzentrat, einem druckfesten und diffusionsdichten Behälter und einer speziellen Feinsprühpumpe mit Vordruckaufbau.

Gegenstand der Erfindung ist daher ein Kompakthaarsprayprodukt wie im Anspruch 1 definiert.

Der Gegenstand der vorliegenden Erfindung ist hervorragend dazu geeignet, um Haarsprayprodukte in kompakter Bauweise, beispielsweise mit einem Volumen von 40 bis 60 ml herzustellen, welche eine Ergiebigkeit aufweisen, die derjenigen von typischen 250-300 ml Aerosol-Haarspraydosen nahekommt.

Unter einem Kompakthaarsprayprodukt im Sinne der Erfindung wird ein Haarsprayprodukt verstanden, welches eine kompaktere Bauweise als ein typisches Aerosol-Haarspray, d.h. ein Füllvolumen von weniger als 250 ml, insbesondere von maximal 150 ml aufweist. Bevorzugte Füllvolumina betragen 10 bis 100 ml, insbesondere 20 bis 80 ml, besonders bevorzugt sind 40 bis 60 ml. Unter einem Haarspraykonzentrat im Sinne der Erfindung wird eine Zusammensetzung verstanden, welche mindestens ein haarfestigendes Polymer in einer Konzentration enthält, welche größer ist als diejenige von üblichen Haarsprays, die typischerweise bei 4 Gew.%, für Haarlacke bei 6 Gew.% liegt. Vorzugsweise ist die Polymerkonzentration größer als 8 Gew.%, besonders bevorzugt größer als 10 Gew.% bezogen auf die Gesamtzusammensetzung. Die maximale Polymerkonzentration ist vorzugsweise 20 Gew.%.

Unter einer Viskosität, die eine gute Versprühbarkeit ermöglicht, wird im Sinne der Erfindung eine Viskosität verstanden, bei der die mittlere Tröpfchengröße der mit einer Feinsprühpumpe Seaquist-Perfect PZ1 / 100 HVT versprühten Zusammensetzung weniger als 100 µm, vorzugsweise weniger als 80 µm beträgt oder bei der der dv(90) Wert maximal 140 µm, vorzugsweise maximal 130 µm beträgt. Die mittlere Tröpfchengröße und die Tröpfchengrößenverteilung können beispielsweise bestimmt werden mit Hilfe eines Partikelmessgerätes auf Basis von Laserstrahlbeugung, z.B. eines Malvern Particle Sizer Messgerätes. Die bevorzugte kinematische Viskosität beträgt maximal 10 mm²/s, besonders bevorzugt maximal 5 mm²/s, gemessen mit einem Rotationsviskosimeter RheoStress 100 der Firma Haake bei einer Temperatur von 25°C und einem Schergefälle von 0,5 bis 1400 s⁻¹.

Geeignete haarfestigende Polymere sind ausgewählt aus synthetischen oder natürlichen bzw. modifizierten natürlichen Polymeren, welche jeweils, nichtionischen, kationischen, anionischen oder amphoteren Charakter haben können. Es kann auch ein Gemisch mehrerer dieser Polymere eingesetzt werden. Unter haarfestigenden Polymeren werden erfindungsgemäß solche Polymere verstanden, die bei Anwendung in 0,01 bis 5%-iger wässriger, alkoholischer oder wässrig-alkoholischer Lösung in der Lage sind, auf dem Haar einen Polymerfilm abzuscheiden und auf diese Weise das Haar zu festigen.

Geeignete synthetische, nichtionische filmbildende, haarfestigende Polymere sind z.B. Homopolymere des Vinylpyrrolidons, Vinylcaprolactams oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akyponine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/Polypropylenglykol-Copolymere, die beispielsweise unter den Handelsbezeichnungen Ucon® der Union Carbide vertrieben werden.

Geeignete natürliche bzw. modifizierte natürliche, filmbildende Polymere mit haarfestigender Wirkung sind zum Beispiel Chitosan mit einem Molekulargewicht von 20.000 bis ca. 5 Millionen g/mol. Desweiteren können verschiedene Saccharidtypen verwendet werden wie Polysaccharide oder Gemische aus Oligo-, Mono- und Disacchariden, welche beispielsweise unter dem Handelsnamen C-PUR® von der Firma Cerestar, Brüssel, vertrieben werden. Weitere geeignete, natürliche Polymere sind chinesisches Balsamharz, Guar bzw. Guarderivate und Cellulosederivate, z.B. Hydroxypropylcellulose mit einem Molekulargewicht von 30.000 bis 50.000 g/mol, welche beispielsweise unter der Handelsbezeichnung Nisso Sl® von der Firma Lehmann & Voss, Hamburg, vertrieben wird. Ein weiteres natürliches Polymer ist Schellack. Schellack kann in neutralisierter, teilneutralisierter oder unneutralisierter Form zum Einsatz kommen.

Geeignete anionische Polymere enthalten Säuregruppen, die mit geeigneten Basen neutralisierbar sind. Die Säuregruppen sind vorzugsweise ausgewählt aus -COOH, - SO₃H, -OSO₃H, -OPO₂H und -OPO₃H₂. Carbonsäuregruppen sind besonders beorzugt. Die Säuregruppen liegen vorzugsweise zu 50 bis 100% in anionischer bzw. neutralisierter Form vor. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin oder Monoethanolamin und Ammoniak, NaOH u.a..

Das haarfestigende anionische Polymer kann ein natürliches oder ein synthetisches Homo- oder Copolymer mit Säuregruppen enthaltenden Monomereinheiten sein, welches gegebenenfalls mit Comonomeren, die keine Säuregruppen enthalten, copolymerisiert ist. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Itaconsäure, Maleinsäure bzw. Maleinsäureanhydrid oder deren Monoester, Fumarsäure oder deren Monoester, Aldehydocarbonsäuren oder Ketocarbonsäuren. Geeignete anionische Polymere sind aber auch anionische Polyurethane.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden, Methacrylamidensowie Copolymere mit Polyethylenoxid. Ein geeignetes natürliches Polymer ist beispielsweise Schellack.

Bevorzugte anionische Polymere sind vernetzte oder unvernetzte Vinylacetat/Crotonsäure Copolymere (INCI-Bezeichnung: VA/Crotonates Copolymer) und Vinylacetat/ Crotonsäure/Polyethylenoxid Copolymere. Ebenso bevorzugt sind partialveresterte Copolymere zwischen Vinylmethylether und Maleinsäureanhydrid, z.B. Methylvinylether/Maleinsäuremonoethyl- oder -monobutylester Copolymer (INCI-Bezeichnungen: Ethylester of PVM/MA Copolymer, Butylester of PVM/MA Copolymer). Weitere geeignete anionische Polymere sind zum Beispiel Copolymere aus Acrylsäure, Alkylacrylaten und N-Alkylacrylamid (INCI-Bezeichnung: Acrylates/Acrylamide Copolymer), insbesondere Acrylsäure/Ethylacrylat/N-t-Butylacrylamid Terpolymere sowie Copolymere aus ein oder mehreren C1-C5-Alkylacrylaten, insbesondere C2-C4-Alkylacrylaten und Acrylsäure oder Methacrylsäure (INCI-Bezeichnung: Acrylates Copolymer), insbesondere Methacrylsäure/Ethylacrylat/Butylacrylat Terpolymere. Weiterhin geeignet sind Terpolymere aus Vinylacetat, Crotonat und Vinylalkanoat, insbesondere Vinylacetat/ Crotonat/Vinylneodecanoat Copolymere oder Vinylacetat/ Crotonat/Vinylpropionat Copolymere(INCI-Bezeichnungen: VA/Crotonates/Vinyl Neodecanoate Copolymer bzw. VA/ Crotonates/Vinyl Propionate Copolymer).

Geeignete amphotere Polymere sind solche, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren bestehend aus Acrylsäure, Methacrylsäure oder deren Ester (INCI-Bezeichnung: Octylacrylamide/ Acrylates/Butylaminoethyl Methacrylate Copolymer), wie sie zum Beispiel unter dem Handelsnamen Amphomer® oder Amphomer® LV-71 der Firma NATIONAL STARCH, USA erhältlich sind. Weitere geeignete amphotere Polymere sind Copolymere, welche gebildet sind aus mindestens einer ersten Monomerart, welche quaternäre Amingruppen aufweist und mindestens einer zweiten Monomerart, welche Säuregruppen aufweist. Beispiele für derartige Copolymere sind Copolymere von Acrylsäure, Methylacrylat und Methacrylamidopropyltrimethylammoniumchlorid (INCI-Bezeichnung: Polyquaternium-47), wie sie beispielswiese von der Firma Calgon unter der Handelsbezeichnung Merquat® 2001 vertrieben werden, Copolymere aus Acrylamidopropyltrimethylammoniumchlorid und Acrylaten, wie sie beispielsweise von der Firma Stockhausen unter der Handelsbezeichnung W 37194 erhältlich sind oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43), wie sie beispielsweise von der Firma Societe Francaise Hoechst unter der Handelsbezeichnung Bozequat® 4000 vertrieben werden. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/ Acrylates Copolymer.

Geeignete Polymere mit basischen Gruppen haben ein Molekulargewicht von vorzugsweise mindestens 50.000 g/mol, besonders bevorzugt von 100.000 bis 6.000.000 g/mol und enthalten stickstoffhaltige Gruppen wie zum Beispiel primäre, sekundäre oder tertiäre Amine. Die basischen Polymere können mit geeigneten, kosmetisch verträglichen Säuren teilweise oder ganz neutralisiert sein und somit in kationischer Form vorliegen. Geeignete Säuren sind z.B. Ameisensäure, Pyrrolidoncarbonsäure, Milchsäure usw. Die basische Gruppe ist entweder in der Polymerkette oder vorzugsweise als Substituent an einem oder mehreren Monomeren enthalten. Das Polymer mit basischen Gruppen kann ein natürliches oder ein synthetisches Homo- oder Copolymer mit aminsubstituierten Monomereinheiten sowie gegebenenfalls mit nichtbasischen Comonomeren sein. Geeignete Polymere mit basischen Gruppen sind zum Beispiel Copolymere von aminsubstituierten Vinylmonomeren und nicht aminsubstituierten Monomeren. Aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise niedere Alkylgruppen wie zum Beispiel C1- bis C7-Alkylgruppen, besonders bevorzugt C1- bis C3-Alkylgruppen sind.

Nicht aminsubstituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Maleinsäureanhydrid, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit kationischen Gruppen enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren Monomeren enthalten sind. Die Ammoniumgruppen enthaltenden Monomere können mit den oben genannten nicht aminsubstituierten Monomeren copolymerisiert sein. Geeignete ammoniumsubstituierte Vinylmonomere sind zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16), quaternisiertes Vinylpyrrolidon/Dimethylaminoethylmethacrylat Copolymer (Polyquaternium-11), Homo- und Copolymere von Dimethyldiallylammoniumchlorid (Polyquaternium-6 und -7), quaternisierte Hydroxyethylcellulose (Polyquaternium-10) oder quaternisierte Guarderivate.

Von den kationischen Polymeren, die in dem erfindungsgemäßen Mittel enthalten sein können, ist zum Beispiel Polyvinylpyrrolidon/Dimethylaminoethylmethacrylat Copolymer geeignet. Weitere kationische Polymere sind beispielsweise das Copolymer aus Polyvinylpyrrolidon und Imidazoliminmethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcaprolactam, quaternierte Ammoniumsalze aus Hydroxyethylcellulose und einem trimethylammonium-substituierten Epoxid und Vinylpyrrolidon/Methacrylamidopropyltrimethylammoniumchlorid Copolymere.

Weitere geeignete haarfestigende Polymere sind Copolymere aus Vinylpyrrolidon, Vinylcaprolactam und Dialkylaminoalkylmethacrylamiden, wobei die Alkylgruppen vorzugsweise aus 1 bis 3 C-Atomen bestehen. Besonders bevorzugt sind Terpolymere aus Vinylpyrrolidon, Vinylcaprolactam und Dimethylaminopropylmethacrylamid (DMAPMA), welches unter dem Namen Aquaflex® SF-40 von ISP vertrieben wird.

Die für das erfindungsgemäße Kompakthaarspray bevorzugten haarfestigenden Polymere sind ausgewählt aus Copolymeren aus Acrylaten und Acrylathydroxyestern, Copolymeren aus Methylvinylether und Alkylmonoestern von Maleinsäure, Copolymeren aus Acryl- oder Methacrylsäure und Acrylsäure- oder Methacrylsäurealkylestern, Copolymeren aus Vinylacetat und Crotonsäure, Copolymeren aus Vinylacetat, Crotonsäure und Vinylalkanoaten, Copolymeren aus Acryl- oder Methacrylsäure und Acrylsäure- oder Methacrylsäurealkylestern und Acryl- oder Methacrylamiden, Polyvinylcaprolactam, Vinylpyrrolidon/Vinylacetat Copolymeren, Copolymeren aus Vinylpyrrolidon, Vinylcaprolactam und Dialkylaminoalkylacryl- oder -methacrylamiden, Schellack, Copolymeren aus Alkylacrylamiden, Alkylaminoalkylmethacrylaten und zwei oder mehr Monomeren bestehend aus Acrylsäure, Methacrylsäure oder deren Ester und Acrylat/Acrylamid Copolymeren und Mischungen der genannten Polymere. Besonders bevorzugt sind dabei die haarfestigenden Polymere ausgewählt aus Acrylat/Acrylamid Copolymeren, Copolymeren aus Alkylacrylamiden, Alkylaminoalkylmethacrylaten und zwei oder mehr Monomeren bestehend aus Acrylsäure, Methacrylsäure oder deren Ester, Copolymeren aus Vinylacetat und Crotonsäure, Copolymeren aus Vinylacetat, Crotonsäure und Vinylalkanoaten jeweils in einer Menge von 8 bis 30 Gew.% und Polyvinylpyrrolidon/ Vinylacetat Copolymeren in einer Menge von größer 10 bis 30 Gew.%.

Das haarfestigende Polymer ist vorzugsweise in einer Menge von größer 4 Gew.%, besonders bevorzugt von größer als 8 bis 30 Gew.%, besonders bevorzugt von größer 10 bis 20 Gew.% enthalten. Das Polymer ist in dem erfindungsgemäßen Lösungsmittelgemisch löslich oder dispergierbar.

Als alkoholisches Lösungsmittel können C1- bis C4-Alkohole, d.h. Methanol, Ethanol, Isopropanol, n-Propanol und Butanol eingesetzt werden, von denen Ethanol und Isopropanol besonders bevorzugt sind. Das alkoholische Lösungsmittel ist in einer Menge von vorzugsweise 20 bis 80 Gew.%, bevorzugt von 40 bis 75 Gew.%, besonders bevorzugt von 50 bis 70 Gew.% enthalten. Ein wesentlicher Bestandteil des erfindungsgemäßen Kompakthaarsprayproduktes ist ein unter Normalbedingungen (20°C, 1013 mbar) flüssiger, organischer Lösungsmittelzusatz, welcher sowohl die Viskosität des Haarspraykonzentrats senkt als auch den Dampfdruck des Konzentrats erhöht. Dieser Lösungsmittelzusatz wird vorzugsweise in einer Menge von 1 bis 50 Gew.%, besonders bevorzugt von 4 bis 40 Gew.%, ganz besonders bevorzugt von 8 bis 30 Gew.% eingesetzt. Geeignete Zusätze sind insbesondere lineare, verzweigte oder cyclische C5-oder C6-Alkane oder deren Gemische, d.h. n-Pentan, Isopentan, Neopentan, n-Hexan oder die verzweigten Hexanisomere. Besonders bevorzugt sind die Pentane, insbesondere n-Pentan. Diese Zusätze verringern die Viskosität der konzentrierten Polymerzusammensetzung. Eine deutlich höhere Polymerkonzentration als üblich ist wiederum erforderlich, um eine kompakte Bauweise überhaupt zu ermöglichen. Aufgrund der erniedrigten Viskosität des Haarspraykonzentrats ergeben sich verringerte Fließwiderstände bei Betätigung der Sprühpumpe, wodurch mehr Energie für die Verstäubung des Konzentrats zur Verfügung steht, was vermutlich die Ursache für eine feinere Verstäubung und die reduzierte Tröpfchengröße ist. Aufgrund der Erniedrigung des Dampfdrucks der Gesamtzusammensetzung verdampft ein Teil des Lösungsmittels spontan bei Austritt aus der Sprayöffnung, was zu einer weiteren Reduzierung der Tröpfchengröße und zu einer besseren Vernebelung des Sprays führt. Der Dampfdruck des organischen Lösungsmittelzusatzes muß also höher sein als derjenige des verwendeten Lösungsmittels aber niedrig genug, um sicherzustellen, dass der Zusatz unter Normalbedingungen in flüssiger Form vorliegt. Bei Verwendung von bei Raumtemperatur gasförmigen Stoffen ist aufgrund des erforderlichen Gasraums keine kompakte Bauweise möglich. Außerdem haben bei Normalbedingungen gasförmige Stoffe wie die üblichen Treibmittel Propan und Butan den Nachteil, dass sie sofort bei Austritt aus der Düse verdampfen und auf dem Haar im wesentlichen nur noch das Konzentrat ankommt, welches aufgrund der Viskosität nicht ausreichend auf dem Haar verläuft.

Ein weiterer, vorzugsweise enthaltener Bestandteil des erfindungsgemäßen Kompakthaarsprayproduktes ist ein trocknungsverzögernder Lösungsmittelzusatz (a4). Ohne diesen Zusatz erfolgt eine zu schnelle Trocknung der versprühten Zusammensetzung auf dem behandelten Haar, es erfolgt nicht in ausreichendem Maße ein Verlaufen der versprühten Zusammensetzung auf dem Haar und es kann zu einem sogenannten Perlenketteneffekt kommen, bei welchem nur punktuelle Polymerablagerungen auf dem Haar beobachtet werden aber keine ausreichende Filmbildung und Vernetzung der Haare erfolgt. Als trocknungsverzögernder Zusatz wird Wasser bevorzugt. Es sind aber auch andere flüssige, die Trocknungszeit verlängernde Stoffe denkbar, beispielsweise mehrwertige Alkohole wie z.B. Glykole wie Ethylenglykol und Propylenglykole oder Glycerin oder auch längerkettige Alkohole. Der Gehalt des trocknungsverzögernden Lösungsmittelzusatzes beträgt vorzugsweise 1 bis 20 Gew.%, besonders bevorzugt 2 bis 16 Gew.%, ganz besonders bevorzugt von 4 bis 12 Gew.%.

Das erfindungsgemäße Haarspraykonzentrat kann weitere, übliche kosmetische Zusatzstoffe enthalten, zum Beispiel Weichmacher wie Glycerin, Glykol, Phtalatester oder Zitronensäureester; Duftstoffe und Parfümöle, Lichtschutzmittel, UV-Filter, haarpflegende Zusätze, Kämmbarkeitsverbesserer, Feuchthaltemittel, Farbstoffe, Korrosionsinhibitoren, Antioxidantien und Konservierungsstoffe in einer Menge von jeweils 0,01 bis 10 Gew.% und einer Gesamtmenge von 0,01 bis 20 Gew.%.

Der Behälter des erfindungsgemäßen Kompakthaarsprayproduktes kann aus einem beliebigen Material gefertigt sein, sofern das Material gegen den auf Grund des dampfdruckerhöhenden organischen Lösungsmittelzusatzes leicht erhöhten Innendruck ausreichend druckfest ist sowie für den organischen Lösungsmittelzusatz ausreichend diffusionsdicht ist. Geeignete Materialien sind zum Beispiel die für Aerosolverpackungen üblicherweise verwendeten Metalle wie Aluminium oder Weißblech. Bevorzugt werden allerdings durchsichtige oder zumindest durchscheinende Materialien, so dass von außen die Produktkonsistenz und/oder die Füllmenge sichtbar ist. Vorzugsweise ist der Produktbehälter im wesentlichen aus Polyethylenterephtalat (PET) gefertigt.

Die für das erfindungsgemäße Kompakthaarsprayprodukt verwendete Sprühpumpe weist einen maximalen Hub von 0,12 ml, vorzugsweise von 0,075 bis 0,115 ml, besonders bevorzugt von 0,08 bis 0,11 ml auf. Weiterhin handelt es sich um eine Sprühpumpe mit einem Vordruckaufbau, d.h. bei Betätigung der Pumpe und vor Abgabe des Sprays baut sich zunächst ein Druck auf. Hierdurch wird ein feineres Zerstäuben und eine weitere Reduzierung der Tröpfchengröße erreicht.

Im Zusammenhang mit der Zusammensetzung des Haarspraykonzentrats sind an das Material, aus dem die Sprühpumpe aufgebaut ist, besondere Anforderungen zu stellen. Die Zylinder üblicher Sprühpumpen für Pumphaarsprays sind aus Kunststoffen wie z.B. Polypropylen, die in Kontakt mit organischen Lösungsmittelzusätzen wie z.B. Pentan quellen können. Eine derartige Standardpumpe versagt nach einiger Zeit. Erfindungsgemäß kommt eine Sprühpumpe zum Einsatz, welche im Zylinderbereich im wesentlichen aus einem gegenüber dem organischen Lösungsmittelzusatz quellresistenten Material gefertigt ist. Ein derartiges Material ist beispielsweise Polyoxymethylen (POM). Eine erfindungsgemäß geeignete Sprühpumpe ist beispielsweise die Feinsprühpumpe Seaquist-Perfect PZ1 / 100 HVT.

Eine Belüftungsöffnung ist im Gegensatz zu üblichen Sprühpumpen nicht erforderlich, da der beim Entleeren entstehende Gasraum durch den nachverdampfenden, leicht flüchtigen organischen Lösungsmittelzusatz gefüllt wird und eine Luftzuführung nicht notwendig ist. Auch wenn das Vorhandensein einer Belüftungsöffnung aufgrund des nur sehr geringen Verlustes des organischen Lösungsmittelzusatzes akzeptabel ist, wird eine Pumpe ohne Belüftungsöffnung bevorzugt.

Das erfindungsgemäße Kompakthaarsprayprodukt zeichnet sich dadurch aus, dass es die Herstellung von Haarsprays in kompakter Bauweise ermöglicht, gegenüber üblichen Pumpsprays einen geringeren Nässeeffekt und kürzere Trocknungszeiten zeigt und in den Anwendungseigenschaften an diejenigen von üblichen Haarsprays herankommt, auf jeden Fall aber als neuartiger Produkttyp zwischen einem klassischen Pumphaarspray und einem klassischen Aerosol-Haarspray angesehen werden kann.

Einige wesentliche Eigenschaften eines typischen, erfindungsgemäßen Kompakthaarsprayproduktes sind in der folgenden Tabelle den Eigenschaften von herkömmlichen Pumphaarsprays sowie herkömmlichen Aerosolhaarsprays gegenübergestellt.

| | Kompakt-haarspray | Pump-haarspray | Aerosol-haarspray |
|---|---|---|---|
| Polymergehalt | 10-20 Gew.% | 3-8 Gew.% | 2-6 Gew.% |
| Packungsvolumen | 60 ml | 150 ml | 250 ml |
| Anwendungsmenge | 1,5 g | 3,5 g | 5,0 g |
| Anwendungen/100 ml | 52 | 24 | 14 |
| Ergiebigkeit | 4-fach | 2-fach | 1-fach |
| Anwendungen/Packung | 31 | 36 | 35 |
| dv(50) | ca. 78 µm | ca. 80 µm | ca. 39 µm |
| Trockenzeit | Gut | Schlecht | Gut |

Das erfindungsgemäße Kompaktspray zeichnet sich gegenüber einem herkömmlichen Pumpspray bei einer rund doppelt so grossen Ergiebigkeit durch ein wesentlich besseres Trocknungsverhalten aus, welches an dasjenige eines Aerosolhaarsprays heranreicht. Gegenüber einem herkömmlichen Aerosolspray ist die Ergiebigkeit sogar rund viermal höher.

Die nachfolgenden Beispiele sollen den Gegenstand der Erfindung näher erläutern.

### Beispiele

### Beispiel 1

| Haarspraykonzentrat: | |
|---|---|
| 20,0 g | Pentan |
| 12,0 g | Acrylates/Acrylamid Copolymer (Ultrahold® 8 von BASF) |
| 5,0 g | Wasser |
| 1,16 g | Aminomethylpropanol |
| 0,45 g | Parfüm |
| 0,36 g | Dimethicone Copolyol |
| 0,36 g | Triethylcitrat |
| ad 100 g | Ethanol |

Das Haarspraykonzentrat wird in einen röhrenförmigen 40 ml Behälter aus durchscheinendem Polyethylenterephtalat gefüllt. Der gefüllte Behälter wird mit einer Feinsprühpumpe vom Typ Seaquist-Perfect PZ1 / 100 HVT mit einem Pumpenhub von 0,1 ml versehen.

### Beispiel 2

| Haarspraykonzentrat: | |
|---|---|
| 20,0 g | Pentan |
| 15,0 g | Acrylates/Acrylamid Copolymer (Ultrahold® 8 von BASF) |
| 5,0 g | Wasser |
| 1,45 g | Aminomethylpropanol |
| 0,45 g | Parfüm |
| 0,45 g | Dimethicone Copolyol |
| 0,45 g | Triethylcitrat |
| ad 100 g | Ethanol |

Das Haarspraykonzentrat wird in einen röhrenförmigen 60 ml Behälter aus durchscheinendem Polyethylenterephtalat gefüllt. Der gefüllte Behälter wird mit einer Feinsprühpumpe vom Typ Seaquist-Perfect PZ1 / 100 HVT mit einem Pumpenhub von 0,1 ml versehen. Es wird etwa die Anwendungszahl einer 250 ml Aerosolspraydose erreicht. Für die Tröpfchengrößeverteilung wurden die folgenden Werte ermittelt:
dv(50) = 78 µm, dv(90) = 109 µm

### Beispiel 3

| Haarspraykonzentrat: | |
|---|---|
| 20,0 g | Pentan |
| 12,0 g | Vinylacetat/Crotonsäure/Vinylneodecanoat Terpolymer (Resyn 28-2930, National Starch) |
| 5,0 g | Wasser |
| 1,24 g | Aminomethylpropanol |
| 0,45 g | Parfüm |
| 0,72 g | Dimethicone Copolyol |
| ad 100 g | Ethanol |

Das Haarspraykonzentrat wird in einen röhrenförmigen 40 ml Behälter aus durchscheinendem Polyethylenterephtalat gefüllt. Der gefüllte Behälter wird mit einer Feinsprühpumpe vom Typ Seaquist-Perfect PZ1 / 100 HVT mit einem Pumpenhub von 0,1 ml versehen.

### Beispiel 4

| Haarspraykonzentrat: | |
|---|---|
| 20,0 g | Pentan |
| 15,0 g | VA/Crotonates Copolymer (Resyn 28-1310, National Starch) |
| 5,0 g | Wasser |
| 1,55 g | Aminomethylpropanol |
| 0,45 g | Parfüm |
| 0,90 g | Triethylcitrat |
| ad 100 g | Ethanol |

Das Haarspraykonzentrat wird in einen röhrenförmigen 60 ml Behälter aus durchscheinendem Polyethylenterephtalat gefüllt. Der gefüllte Behälter wird mit einer Feinsprühpumpe vom Typ Seaquist-Perfect PZ1 / 100 HVT mit einem Pumpenhub von 0,1 ml versehen.Es wird etwa die Anwendungszahl einer 250 ml Aerosolspraydose erreicht.

## Patentansprüche

1. Kompakthaarsprayprodukt bestehend aus
(A) einem Haarspraykonzentrat, welches eine viskosität aufweist, die eine gute Versprühbarkeit ermöglicht, d.h. eine Viskosität, bei der die mittlere Tröpfchengröße weniger als 100 µm beträgt, wenn die Zusammensetzung mit einer Feinsprühpumpe Seaquist-Perfect PZ1/100 HVT versprüht wird, und mit einem Gehalt an
(a1) größer 4 Gew.% bezogen auf das Haarspraykonzentrat (A) mindestens eines haarfestigenden Polymers,
(a2) einem alkoholischen Lösungsmittel in welchem das Polymer (a1) löslich oder dispergierbar ist,
(a3) mindestens einem die Viskosität des Konzentrats senkenden und den Dampfdruck des Konzentrats erhöhenden, unter Normalbedingungen, d.h. 20°C, 1013 mbar, flüssigen organischen Lösungsmittelzusatz,
(B) einem druckfesten und gegenüber dem Konzentrat (A) diffusionsdichten Behälter mit einem Füllvolumen von weniger als 250 ml und
(C) einer Feinsprühpumpe mit
(c1) einem Zylinderbereich aus einem gegenüber dem organischen Lösungsmittelzusatz (a3) quellresistenten Material,
(c2) einem maximalen Hub von 0,12 ml und
(c3) einem Vordruckaufbau.

2. Kompakthaarsprayprodukt nach Anspruch 1, **dadurch gekennzeichnet, dass** die Konzentration an haarfestigenden Polymeren (a1) mehr als 10 Gew.% bezogen auf das Haarspraykonzentrat (A) beträgt.

3. Kompakthaarsprayprodukt nach Anspruch 1 oder 2, **dadurch gekennzeichnet, dass** die haarfestigenden Polymere ausgewählt sind aus Copolymeren aus Acrylaten und Acrylathydroxyestern, Copolymeren aus Methylvinylether und Alkylmonoestern von Maleinsäure, Copolymeren aus Acryloder Methacrylsäure und Acrylsäure- oder Methacrylsäurealkylestern, Copolymeren aus Vinylacetat und Crotonsäure, Copolymeren aus Vinylacetat, Crotonsäure und Vinylalkanoaten, Copolymeren aus Acryl- oder Methacrylsäure und Acrylsäure- oder Methacrylsäurealkylestern und Acryl- oder Methacrylamiden, Polyvinylcaprolactam, Vinylpyrrolidon/Vinylacetat Copolymeren, Copolymeren aus Vinylpyrrolidon, Vinylcaprolactam und Dialkylaminoalkylacryl- oder -methacrylamiden, Schellack, Copolymeren aus Alkylacrylamiden, Alkylaminoalkylmethacrylaten und zwei oder mehr Monomeren bestehend aus Acrylsäure, Methacrylsäure oder deren Ester und Acrylat/Acrylamid Copolymeren und Mischungen der genannten Polymere.

4. Kompakthaarsprayprodukt nach Anspruch 3, **dadurch gekennzeichnet, dass** die haarfestigenden Polymere ausgewählt sind aus Acrylat/Acrylamid Copolymeren, Copolymeren aus Alkylacrylamiden, Alkylaminoalkylmethacrylaten und zwei oder mehr Monomeren bestehend aus Acrylsäure, Methacrylsäure oder deren Ester, Copolymeren aus Vinylacetat und Crotonsäure, Copolymeren aus Vinylacetat, Crotonsäure und Vinylalkanoaten jeweils in einer Menge von 8 bis 30 Gew.% und Polyvinylpyrrolidon/Vinylacetat Copolymeren in einer Menge von größer 10 bis 30 Gew.%.

5. Kompakthaarsprayprodukt nach einem der vorgenannten Ansprüche, **dadurch gekennzeichnet, dass** das alkoholische Lösungsmittel (a2) ausgewählt ist aus einwertigen C1-bis C4-Alkoholen und deren Gemischen.

6. Kompakthaarsprayprodukt nach einem der vorgenannten Ansprüche, **dadurch gekennzeichnet, dass** der organische Lösungsmittelzusatz (a3) ausgewählt ist aus linearen, verzweigten oder cyclischen C5- bis C6-Kohlenwasserstoffen und deren Gemischen.

7. Kompakthaarsprayprodukt nach Anspruch 6, **dadurch gekennzeichnet, dass** der organische Lösungsmittelzusatz (a3) ausgewählt ist aus n-Pentan, Isopentan und Neopentan und deren Gemischen.

8. Kompakthaarsprayprodukt nach einem der vorgenannten Ansprüche, **dadurch gekennzeichnet, dass** das Haarspraykonzentrat zusätzlich einen trocknungsverzögernden Lösungsmittelzusatz (a4) enthält.

9. Kompakthaarsprayprodukt nach Anspruch 8, **dadurch gekennzeichnet, dass** der trocknungsverzögernde Lösungsmittelzusatz (a4) Wasser ist.

10. Kompakthaarsprayprodukt nach einem der vorgenannten Ansprüche, **dadurch gekennzeichnet, dass** der druckfeste und diffusionsdichte Behälter (B) im wesentlichen aus einem durchsichtigen oder durchscheinendem Material ist.

11. Kompakthaarsprayprodukt nach Anspruch 10, **dadurch gekennzeichnet, dass** der druckfeste und diffusionsdichte Behälter (B) im wesentlichen aus Polyethylenterephtalat ist.

12. Kompakthaarsprayprodukt nach einem der vorgenannten Ansprüche, **dadurch gekennzeichnet, dass** der Zylinderbereich der Feinsprühpumpe mit Vordruckaufbau (C) Polyoxymethylen als quellrestistentes Material enthält.

13. Kompakthaarsprayprodukt nach einem der vorgenannten Ansprüche, **dadurch gekennzeichnet, dass** der Hub der Feinsprühpumpe mit Vordruckaufbau (C) maximal 0,11 ml beträgt.

## Claims

1. Compact hairspray product consisting of
(A) a hairspray concentrate which has a viscosity which permits good sprayability, i.e. a viscosity at which the average particle size is less than 100 µm when the composition is sprayed using a Seaquist-Perfect PZ1/100 HVT fine spray pump, and with a content of
(a1) greater than 4% by weight, based on the hairspray concentrate (A), of at least one hair-setting polymer,
(a2) an alcoholic solvent in which the polymer (a1) is soluble or dispersible,
(a3) at least one organic solvent additive which reduces the viscosity of the concentrate and increases the vapour pressure of the concentrate and is liquid under normal conditions, i.e. 20°C, 1 013 mbar,
(B) a pressure-resistant container which is diffusion-tight to the concentrate (A) and has a fill volume of less than 250 ml and
(C) a fine spray pump with
(c1) a cylinder region made of a material which is swell-resistant to the organic solvent additive (a3),
(c2) a maximum stroke of 0.12 ml and
(c3) a preliminary pressure build-up.

2. Compact hairspray product according to Claim 1, **characterized in that** the concentration of hair-setting polymers (a1) is more than 10% by weight, based on the hairspray concentrate (A).

3. Compact hairspray product according to Claim 1 or 2, **characterized in that** the hair-setting polymers are chosen from copolymers of acrylates and acrylate hydroxy esters, copolymers of methyl vinyl ether and alkyl monoesters of maleic acid, copolymers of acrylic acid or methacrylic acid and acrylic or methacrylic alkyl esters, copolymers of vinyl acetate and crotonic acid, copolymers of vinyl acetate, crotonic acid and vinyl alkanoates, copolymers of acrylic acid or methacrylic acid and acrylic or methacrylic alkyl esters and acrylamides or methacrylamides, polyvinylcaprolactam, vinylpyrrolidone/vinyl acetate copolymers, copolymers of vinylpyrrolidone, vinylcaprolactam and dialkylaminoalkylacryl- or -methacrylamides, shellac, copolymers of alkylacrylamides, alkylaminoalkyl methacrylates and two or more monomers consisting of acrylic acid, methacrylic acid or esters thereof and acrylate/acrylamide copolymers and mixtures of said polymers.

4. Compact hairspray product according to Claim 3, **characterized in that** the hair-setting polymers are chosen from acrylate/acrylamide copolymers, copolymers of alkylacrylamides, alkylaminoalkyl methacrylates and two or more monomers consisting of acrylic acid, methacrylic acid or esters thereof, copolymers of vinyl acetate and crotonic acid, copolymers of vinyl acetate, crotonic acid and vinyl alkanoates, in each case in an amount of from 8 to 30% by weight and polyvinylpyrrolidone/ vinyl acetate copolymers in an amount greater than 10 to 30% by weight.

5. Compact hairspray product according to one of the preceding claims, **characterized in that** the alcoholic solvent (a2) is chosen from monohydric C₁- to C₄-alcohols and mixtures thereof.

6. Compact hairspray product according to one of the preceding claims, **characterized in that** the organic solvent additive (a3) is chosen from linear, branched or cyclic C₅- to C₆-hydrocarbons and mixtures thereof.

7. Compact hairspray product according to Claim 6, **characterized in that** the organic solvent additive (a3) is chosen from n-pentane, isopentane and neopentane and mixtures thereof.

8. Compact hairspray product according to one of the preceding claims, **characterized in that** the hairspray concentrate additionally comprises a drying-delaying solvent additive (a4).

9. Compact hairspray product according to Claim 8, **characterized in that** the drying-delaying solvent additive (a4) is water.

10. Compact hairspray product according to one of the preceding claims, **characterized in that** the pressure-resistant and diffusion-tight container (B) is essentially made of a transparent or translucent material.

11. Compact hairspray product according to Claim 10, **characterized in that** the pressure-resistant and diffusion-tight container (B) is essentially made of polyethylene terephthalate.

12. Compact hairspray product according to one of the preceding claims, **characterized in that** the cylinder region of the fine spray pump with preliminary pressure build-up (C) comprises polyoxymethylene as swell-resistant material.

13. Compact hairspray product according to one of the preceding claims, **characterized in that** the stroke of the fine spray pump with preliminary pressure build-up (C) is at most 0.11 ml.

## Revendications

1. Produit compact de laque pour cheveux, constitué de
(A) un concentré de laque pour cheveux présentant une viscosité qui permet une bonne aptitude à la pulvérisation, c'est-à-dire une viscosité à laquelle la taille de gouttelettes moyenne est inférieure à 100 µm, lorsque la composition est pulvérisée avec une pompe de pulvérisation fine Seaquist-Perfect PZ1/100 HVT, et contenant
(a1) plus de 4 % en poids, par rapport au concentré de laque pour cheveux (A), d'au moins un polymère fixateur des cheveux,
(a2) un solvant alcoolique dans lequel le polymère (a1) est soluble ou dispersable,
(a3) au moins un additif solvant organique réduisant la viscosité du concentré et augmentant la pression de vapeur du concentré, et liquide dans des conditions normales, c'est-à-dire à 20°C et 1 013 mbar,
(B) un récipient résistant à la pression et étanche à la diffusion vis-à-vis du concentré (A), avec un volume de remplissage inférieur à 250 ml, et
(C) une pompe de pulvérisation fine avec
(c1) une zone de cylindre en un matériau résistant au gonflage vis-à-vis de l'additif solvant organique (a3),
(c2) une course maximale de 0,12 ml et
(c3) une montée en pression préliminaire.

2. Produit compact de laque pour cheveux selon la revendication 1, **caractérisé en ce que** la concentration en polymères fixateurs des cheveux (a1) est supérieure à 10 % en poids, par rapport au concentré de laque pour cheveux (A).

3. Produit compact de laque pour cheveux selon la revendication 1 ou 2, **caractérisé en ce que** les polymères fixateurs des cheveux sont choisis parmi des copolymères d'acrylates et d'hydroxyesters d'acrylates, des copolymères d'éther méthylvinylique et de monoesters alkyliques de l'acide maléique, des copolymères d'acide acrylique ou méthacrylique ou d'esters alkyliques de l'acide acrylique ou de l'acide méthacrylique, des copolymères d'acétate de vinyle et d'acide crotonique, des copolymères d'acétate de vinyle, d'acide crotonique et d'alcanoates de vinyle, des copolymères d'acide acrylique ou méthacrylique et d'esters alkyliques de l'acide acrylique ou de l'acide méthacrylique et d'acrylamides ou de méthacrylamides, le polyvinylcaprolactame, des copolymères vinylpyrrolidone/acétate de vinyle, des copolymères de vinylpyrrolidone, de vinylcaprolactame et de dialkylaminoalkylacryl- ou - méthacrylamides, la gomme-laque, des copolymères d'alkylacrylamides, de méthacrylates d'alkylaminoalkyle et de deux monomères ou plus constitués de l'acide acrylique, de l'acide méthacrylique ou de leurs esters et des copolymères acrylate/acrylamide, et des mélanges desdits polymères.

4. Produit compact de laque pour cheveux selon la revendication 3, **caractérisé en ce que** les polymères fixateurs des cheveux sont choisis parmi des copolymères acrylate/acrylamide, des copolymères d'alkylacrylamides, de méthacrylates d'alkylaminoalkyle et de deux monomères ou plus constitués de l'acide acrylique, de l'acide méthacrylique et de leurs esters, des copolymères d'acétate de vinyle et d'acide crotonique, des copolymères d'acétate de vinyle, d'acide crotonique et d'alcanoates de vinyle, dans chaque cas en une quantité de 8 à 30 % en poids, et des copolymères polyvinylpyrrolidone/acétate de vinyle en une quantité supérieure à 10 à 30 % en poids.

5. Produit compact de laque pour cheveux selon l'une quelconque des revendications précédentes, **caractérisé en ce que** le solvant alcoolique (a2) est choisi parmi des alcools en C₁ à C₄ et leurs mélanges.

6. Produit compact de laque pour cheveux selon l'une quelconque des revendications précédentes, **caractérisé en ce que** l'additif solvant organique (a3) est choisi parmi des hydrocarbures en C₅ à C₆ linéaires, ramifiés ou cycliques et leurs mélanges.

7. Produit compact de laque pour cheveux selon la revendication 6, **caractérisé en ce que** l'additif solvant organique (a3) est choisi parmi le n-pentane, l'isopentane et le néopentane, et leurs mélanges.

8. Produit compact de laque pour cheveux selon l'une quelconque des revendications précédentes, **caractérisé en ce que** le concentré de laque pour cheveux comprend en outre un additif solvant retardant le séchage (a4).

9. Produit compact de laque pour cheveux selon la revendication 8, **caractérisé en ce que** l'additif solvant retardant le séchage (a4) est l'eau.

10. Produit compact de laque pour cheveux selon l'une quelconque des revendications précédentes, **caractérisé en ce que** le récipient résistant à la pression et étanche à la diffusion (B) est essentiellement composé d'un matériau transparent ou translucide.

11. Produit compact de laque pour cheveux selon la revendication 10, **caractérisé en ce que** le récipient résistant à la pression et étanche à la diffusion (B) est essentiellement en polyéthylène-téréphtalate.

12. Produit compact de laque pour cheveux selon l'une quelconque des revendications précédentes, **caractérisé en ce que** la zone de cylindre de la pompe de pulvérisation fine avec une montée en pression préliminaire (C) comprend du polyoxyméthylène en tant que matériau résistant au gonflage.

13. Produit compact de laque pour cheveux selon l'une quelconque des revendications précédentes, **caractérisé en ce que** la course de la pompe de pulvérisation fine avec une montée en pression préliminaire (C) est d'au plus 0,11 ml.
